# EUROPEAN PATENT SPECIFICATION

(11) **EP 4 410 250 B1**
(45) Date of publication and mention of the grant of the patent: **07.05.2025**
(21) Application number: 24154611.8
(22) Date of filing: 30.01.2024
(51) Int. Cl.: A61F 5/02

(54) **ORTHOPAEDIC CORRECTIVE CORSET**
ORTHOPÄDISCHES KORREKTURKORSETT
CORSET DE CORRECTION ORTHOPÉDIQUE

(30) Priority: 02.02.2023 IT 202300001710
(43) Date of publication of application: 07.08.2024
(73) Proprietor: Postura e Sport S.r.l., 43037 Lesignano de' Bagni (PR) (IT)
(72) Inventor: CANALI, Vincenzo, 43037 Lesignano de' Bagni (PR) (IT)
(74) Representative: Studio Torta S.p.A.

(56) References cited:
- EP-A1- 1 983 947
- EP-A1- 3 052 061
- US-A1- 2016 045 387

## Description

### Technical field

The invention relates to an orthopaedic corrective corset.

More in detail, the invention relates to a corset aimed at correcting postural problems or at correcting pathological deviations of the spine, such as for example scoliosis.

### State of the art

The use of corrective corsets for treating pathological deviations the spine is a known practice. The corset is normally used to correct wrong postural behaviours in order to reduce pathological deviations, thus avoiding, as much as possible, surgeries.

The corsets commonly used for the correction of scoliosis are characterized by particularly large-sized and uncomfortable structures and are configured to exert a thrust upon the spine suffering from the pathology at the back. The rear thrusting action is often obtained by means of metal upright rods or by means of (metal or plastic) plates, which act upon portions of the dorsal area where the malformations are located. An example of a corset of this type is disclosed in document EP1983947, disclosing an orthopaedic corrective corset according to the preamble of claim 1.

However, corrective corsets of this type still have small percentages of success in terms of reduction of the deviations, especially in the most serious cases that are affected by severe spine deviations. As a matter of fact, the use of this type of corsets often does not avoid surgeries, which are invasive and involve significant risks for the patient.

### Description of the invention

The object of the invention is to provide an orthopaedic corrective corset that does not suffer from the drawbacks of the prior art discussed above; in particular, the object of the invention is to provide a corrective corset which is more effective, compared to known solutions, in reducing pathological deviations of the spine and, at the same time, is easy and comfortable to be applied.

According to these objects, the invention relates to an orthopaedic corrective corset for the correction of pathological deviations of a patient's spine; the corset comprising two shells, designed to be arranged along the respective sides of the patient's torso, and a connecting device, designed to connect the shells to each other;
each shell being provided with a front portion, which, in use, extends from the respective side along the front portion of the patient's torso, and with a rear portion, which, in use, extends from the respective side along the rear portion of the patient's torso;
the rear portions of the shells are shaped so as to leave, in use, a dorsal band including the spine free;
each front portion of each shell being shaped so as to subtend at least a respective thoracic portion of the patient's torso and being provided with at least one thrust device arranged and configured to exert a thrusting action upon said thoracic portion.

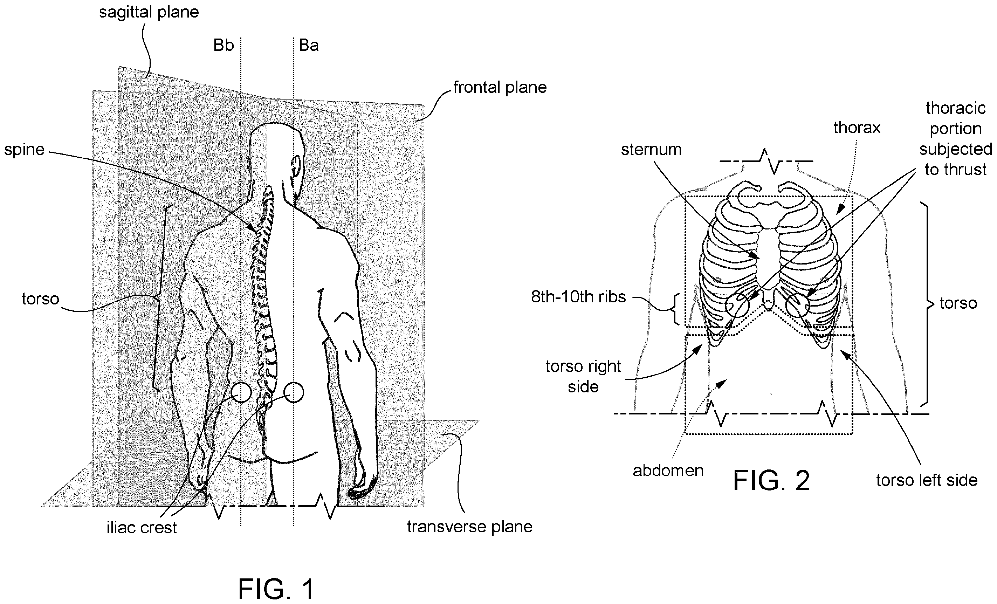

The corset according to the invention basically exerts, thanks to the shells acting upon the sides of the patient's torso, a lateral corrective action (along the frontal plane - see figure 1) and, thanks to the presence of the thrust devices acting upon respective thoracic portions, a front corrective action (along the sagittal plane).

Therefore, the corset according to the invention carries out an exclusively anterolateral spine alignment thrusting action without performing any thrust upon the rear torso and, especially, without performing any thrust upon the dorsal area of the rear torso including the spine.

This solution is designed to obtain very significant levels of correction of pathological deviations in short times. The corrective action of the corset according to the invention, even though it has to be accompanied by postural exercises aimed at strengthening abdomen and hamstring muscles, such as hip extensors and glenohumeral joint fixator muscles, would allow for significant improvements in very short times, even in case of extremely severe deviations of the spine (exceeding 30°).

The anterolateral thrusting action defined by the corset according to the invention manages to force the patient to assume a re-alignment postural behaviour that "trains" abdominal muscles and stabilizes them (thanks to the thrust device that acts at the front) so as to counter the excess of compensation actions needed during natural torsions of the spine that take place when the patient walks.

While walking and, in particular, while changing the feet resting on the ground, the torso oscillates on three planes (sagittal plane, transverse plane, frontal plane) and the spine is subjected to continuous torsions that, in non-pathological subjects, are compensated by the action of the abdominal muscles activated by the retraction/lowering of the rib cage which, by stabilizing the dorsolumbar spine, avoids an increase in the lever arm involved in the torsion/rotation.

In pathological subjects, the abdominal compensation actions are lacking and the spine, with every torsion, increasingly yields, thus determining deviations of the spine on the frontal and transverse plane.

### List of the drawings

Further features and advantages of the invention will be best understood upon perusal of the following description of a non-limiting embodiment thereof, with reference to the accompanying drawings, wherein:
- figure 1 is a schematic rear perspective view of a patient's torso, in which some anatomy portions are represented as transparent and some parts are left out for greater clarity;
- figure 2 is a schematic front view of a patient's torso, in which some anatomy parts are represented as transparent and some parts are left out for greater clarity;
- figure 3 shows a schematic perspective view, with parts left out for greater clarity, of the orthopaedic corrective corset according to the invention in a first operating configuration;
- figure 4 shows a schematic view from the top, with parts left out for greater clarity, of the orthopaedic corrective corset of figure 3;
- figure 5 shows a cross-section view, along plane V-V, of the corset of figure 3, with an enlarged portion and with parts left out for greater clarity;
- figures 6-8 schematically show the use of the corset according to the invention in different configurations for the correction of some types of spine deviations.

### Description of an embodiment of the invention

Figure 1 and figure 2 show a patient's torso at the back and at the front, respectively.

Figures 1 and 2 identify those anatomy elements deemed useful for understanding the use and the main features of the orthopaedic corrective corset according to the invention, as discussed more in detail below.

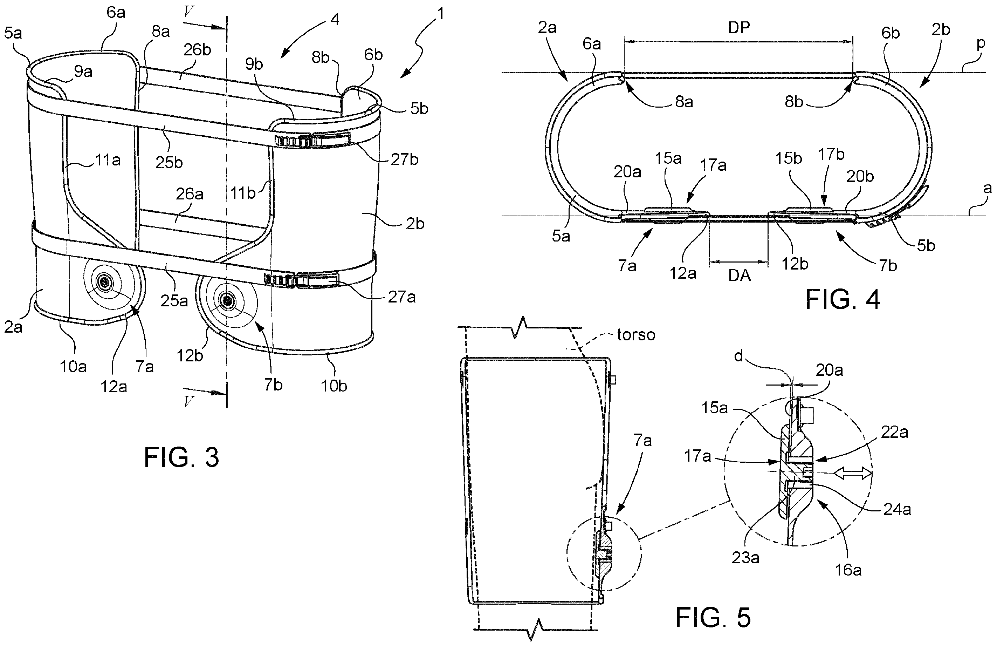

In figure 3, reference number 1 indicates, as a whole, an orthopaedic corrective corset according to the invention in a first operating configuration.

The corset 1 is aimed at correcting pathological deviations of the spine of the patient on which it is applied.

The corset 1 comprises two shells 2a, 2b, designed to be arranged along the respective (right and left) sides of the patient's torso, and a connecting device 4, designed to connect the shells 2a, 2b to each other.

Each shell 2a, 2b is provided with a front portion 5a, 5b, which, in use, extends from the respective (right and left) side along the front portion of the patient's torso, and with a rear portion 6a, 6b, which, in use, extends from the respective side along the rear portion of the patient's torso.

The rear portions 6a, 6b are shaped so as to leave, in use, a dorsal band including the spine free.

In other words, the rear portions 6a, 6b extend along the rear portion of the patient's torso in a limited manner. In particular, the rear portions 6a, 6b are preferably configured to extend from the respective side along the rear portion of the patient's torso without crossing over a respective vertical axis Ba, Bb going through the iliac crest proximal to the side along which the shell 2a, 2b is arranged.

In other words, the rear portions 6a, 6b are provided with respective terminal edges 8a, 8b, which do not cross over the respective vertical axis Ba, Bb going through the iliac crest proximal to the side along which the shell 2a, 2b is arranged.

Between the rear portions 6a, 6b there are no further components of the corset 1 having a corrective function (except for the parts defining the connecting device). In other words, the corset 1 is not configured to act upon the spine at the back.

Each front portion 5a, 5b of each shell 2a, 2b is shaped so as to subtend at least a respective thoracic portion of the patient's torso and is provided with at least one thrust device 7a, 7b arranged and configured to exert a thrusting action upon said thoracic portion.

With reference to figure 4, the shells 2a, 2b substantially are C-shaped and, in use, are arranged so as to face one another with the concavities facing each other.

With reference to figure 3 and figure 4, the front portion 5a, 5b of each shell 2a, 2b is provided with a first edge 9a, 9b, with a second edge 10a, 10b and with a terminal edge 11a, 11b extending between the first edge 9a, 9b and the second edge 10a, 10b. Each terminal edge 11a, 11b is preferably provided with an appendix 12a, 12b provided with the respective thrust device 7a, 7b and is configured so as to subtend at least the thoracic portion upon which the thrust device 7a, 7b acts.

The shells 2a, 2b are preferably sized so that the rear terminal portions comprising the terminal edge 11a, 11b substantially extend along a front plane a and so that the rear terminal portions comprising the terminal edge 8a, 8b substantially extend along a rear plane p.

In use, the terminal edge 11a, 11b preferably is substantially vertical, whereas the appendix 12a, 12b is defined by a prolongation of the front position 5a, 5b and substantially extends on the front plane a (well visible in figure 4).

This solution leads to a configuration of the front portion 5a, 5b of each shell 2a, 2b that is particularly comfortable for the patient, since the terminal edge 11a, 11b extends along the front portion of the patient's torso as little as possible and the sole appendix 12a, 12b reaches the thoracic portion upon which the thrust needs to be exerted.

According to a less comfortable variant which is not shown herein, the terminal edge 11a, 11b extends up to entirely covering the thoracic portion upon which the thrust needs to be exerted.

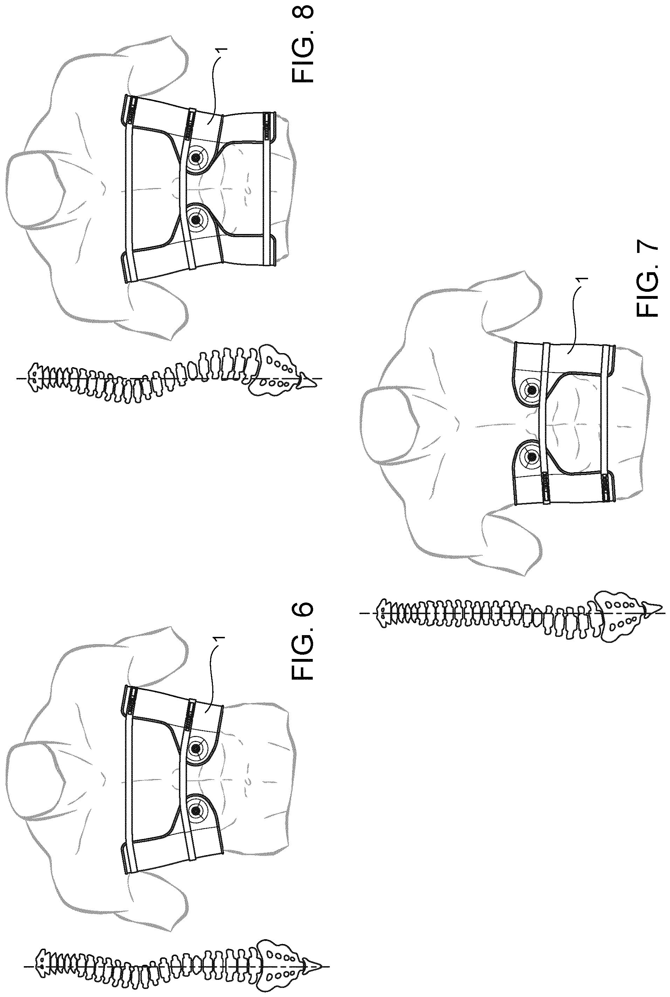

In use, the appendix 12a, 12b is arranged proximal to the second edge 10a, 10b, when the corset 1 is arranged along the patient's sides at a thoracic height for the correction of thoracic scoliosis (see the configuration of figures 3, 4, 5, 6), or is arranged proximal to the first edge 9a, 9b, when the corset 1 is arranged along the patient's sides at an abdominal height for the correction of lumbar scoliosis (see the configuration of figure 7), or is arranged in a substantially central position, when the corset is arranged along the patient's sides along the lumbar and thoracic area for the correction of combined scoliosis (see the configuration of figure 8).

The thoracic portion upon which the thrust device 7a, 7b acts is preferably arranged laterally with respect to the body of the sternum.

More preferably, the thoracic portion upon which the thrust device acts is comprised between the eighth and the tenth rib under the pectoral muscles (see the circular area shown in figure 2).

With reference to figure 5, each thrust device 7a, 7b (only one of them being visible in figure 5) comprises a contact body 15a, 15b, designed to be arranged, in use, in contact with the respective thoracic portion, and an adjusting device 16a, 16b, configured to adjust the thrust exerted by the contact body 15a, 15b upon the respective thoracic portion of the patient.

The contact body 15a, 15b preferably is planar and is provided with a contact face 17a, 17b, which, in use, is in contact with the patient's thoracic portion.

The adjusting device 16a, 16b is configured to adjust the distance d of the contact body 15a, 15b from an inner face 20a, 20b of the front portion 5a, 5b of each shell 2a, 2b.

In this way, the thrust exerted by the contact body 15a, 15b upon the respective thoracic portion, which it subtends, can be adjusted.

The adjusting device 16a, 16b is preferably housed in a through hole 22a, 22b of the front portion 5a, 5b of each shell 2a, 2b.

The adjusting device 16a, 16b comprises a connecting pin 23a, 23b, which is fixed to the contact body 15a, 15b and is threaded on the outside, and a bushing 24a, 24b, which is threaded on the inside and is housed in the respective through hole 22a, 22b.

Hence, the connecting pin 23a, 23b has an end connected to the contact body 15a, 15b and a free end, preferably shaped so as to allow the connecting pin 23a, 23b to be grabbed and rotated by means of a suitable tool (screwdriver, hex key, etc.).

In use, the rotation of the connecting pin 23a, 23b operated by means of a tool in both directions determines a forward or backward movement of the contact body with respect to the thoracic portion. The forward movement of the contact body towards the respective thoracic portion determines an increase in the distance d, whereas the backward movement determines a decrease in the distance d. In the position of minimum thrust, the distance d is 0.

The connecting device 4 is configured to connect the shells 2a, 2b so as to limit their mutual distancing.

With reference to figures 3 and 4 again, the connecting device 4 is configured to connect the shells 2a, 2b so that the front portions 5a, 5b are arranged at a given front distance DA and the rear portions 6a, 6b are arranged at a given rear distance DP.

The connecting device 4 is preferably configured so that the front distance DA between the front portions 5a, 5b of the shells is adjustable and/or the rear distance DP between the rear portions 6a, 6b of the shells is adjustable.

The connecting device 4 preferably comprises at least a front connecting strap 25 provided with a front length adjustment system 26 and a rear connecting strap 27 provided with a rear length adjustment system 28.

The connecting device 4 (in the examples of figures 3-7) preferably comprises two front connecting straps 25a, 25b, respectively arranged in the area of an upper and lower portion of the front portions 5a, 5b of the shells 2a, 2b and respectively provided with a front length adjustment system 27a, 27b, and two rear connecting straps 26a, 26b, respectively arranged in the area of an upper and a lower portion of the rear portions 6a, 6b of the shells 2a, 2b and respectively provided with a rear length adjustment system 28a, 28b (not clearly visible in the accompanying figures).

The front adjustment system 27a, 27b and the rear adjustment system 28a, 28b are, in the non-limiting example described and shown herein, lever adjustment systems.

The front adjustment system 27a, 27b and the rear adjustment system 28a, 28b preferably are lever adjustment systems of the known kind and comprising a toothed portion, provided with a plurality of teeth, and an articulated hook or an adjustable-lever hook, which is configured to hook the toothed portion.

The number of straps used can obviously be greater than two, both at the front and at the back. The configuration of figure 8, for example, uses at least three front straps and at least three rear straps.

Other types of adjusting adjustment systems can obviously be used to obtain the same result.

In this way, the adjustment device 4 is capable of adjusting the front distance DA and the rear distance DP in an independent manner and on two levels (lower, upper and, if necessary, intermediate level). This possibility allows users to properly adjust the lateral and transverse thrust and to obtain a traction, even an asymmetric one, exerted by the shells 2a, 2b so as to force, in the patient, a postural behaviour correcting the spine deviation.

Finally, the apparatus and the method described herein can evidently be subjected to changes and variations, without for this reason going beyond the scope of protection of the appended claims.

## Claims

1. An orthopaedic corrective corset for the correction of pathological deviations of a patient's spine; the corset (1) comprises two shells (2a, 2b), designed to be arranged along the respective sides of the patient's torso, and a connecting device (4), designed to connect the shells (2a, 2b) to each other;
each shell (2a, 2b) being provided with a front portion (5a, 5b), which, in use, extends from the respective side along the front portion of the patient's torso, and a rear portion (6a, 6b), which, in use, extends from the respective side along the rear portion of the patient's torso;
the rear portions (6a, 6b) of the shells (2a, 2b) are shaped in such a way as to leave, in use, a dorsal band including the vertebral column free;
each front portion (5a, 5b) of each shell (2a, 2b) being shaped so as to subtend at least a respective thoracic portion of the patient's torso **characterized in that** each front portion is provided with at least one thrust device (7a, 7b) arranged and configured to exert a thrusting action on said thoracic portion.

2. Corset according to claim 1, wherein the thoracic portion on which the thrust device (7a, 7b) acts is arranged laterally with respect to the body of the sternum.

3. Corset according to claim 2, wherein the thoracic portion on which the thrust device (7a, 7b) acts is between the eighth and tenth rib.

4. Corset according to any of the preceding claims, wherein each thrust device (7a, 7b) comprises a contact body (15a, 15b), suitable to be arranged, in use, in contact with the respective thoracic portion, and an adjusting device (16a, 16b), configured to adjust the thrust exerted by the contact body (15a, 15b) on the respective thoracic portion of the patient.

5. Corset according to claim 4, wherein the adjusting device (16a, 16b) is configured to adjust the position of the contact body (15a, 15b) with respect to an inner face (20a, 20b) of the front portion (5a, 5b) of the shell (2a, 2b).

6. Corset according to claim 5, wherein the adjusting device (16a, 16b) is housed in a through-hole (22a, 22b) of the front portion (5a, 5b) of the shell (2a, 2b).

7. Corset according to any of the preceding claims, wherein the front portion (5a, 5b) of each shell (2a, 2b) is provided with a first edge (9a, 9b), a second edge (10a, 10b) and a terminal edge (11a, 11b) extending between the first edge (9a, 9b) and the second edge (10a, 10b); the terminal edge (11a, 11b) being provided with an appendix (12a, 12b) provided with the respective thrust device (7a, 7b) and configured so as to subtend at least the thoracic portion upon which the thrust device (7a, 7b) acts.

8. Corset according to claim 7, wherein the appendix (12a, 12b) is arranged proximal to the second edge (10a, 10b) when the corset (1) is arranged along the patient's sides at thoracic height for correction of thoracic scoliosis or is disposed proximal to the first edge (9a, 9b) when the corset (1) is arranged along the patient's sides at abdominal height for the correction of lumbar scoliosis or is arranged substantially central between the first edge (9a, 9b) and the second edge (10a, 10b) when the corset (1) is arranged along the patient's sides along the lumbar and thoracic area for the correction of combined scoliosis.

9. Corset according to any of the preceding claims, wherein the rear portion (6a, 6b) of each shell (2a, 2b) extends from the respective side along the rear portion of the patient's torso without crossing over a vertical axis (Ba, Bb) passing through the iliac crest proximal to the side along which the shell (2a, 2b) is arranged.

10. Corset according to any of the preceding claims, wherein the connecting device (4) is configured to connect the shells (2a, 2b) in such a way as to limit their mutual distancing.

11. Corset according to any of the preceding claims, wherein the connecting device (4) is configured to connect the shells such that the front portions (5a, 5b) are arranged at a certain front distance (DA) and the rear portions (6a, 6b) are arranged at a certain rear distance (DP).

12. Corset according to claim 11, wherein the connecting device (4) is configured such that the front distance (DA) between the front portions (5a, 5b) of the shells (2a, 2b) is adjustable and/or the rear distance (DP) between the rear portions (6a, 6b) of the shells (2a, 2b) is adjustable.

13. Corset according to claim 12, wherein the connecting device (4) comprises at least one front connecting strap (25) provided with a front length adjustment system (26) and a rear connecting strap (27) provided with a rear length adjustment system (28).

14. Corset according to claim 13, wherein the connecting device (4) comprises at least two front connecting straps (25a; 25b), respectively arranged at an upper and lower portion of the front portions (5a; 5b) of the shells (2a; 2b) and respectively provided with a front length adjustment system (27a; 27b), and/or at least two rear connecting straps (26a; 26b), respectively arranged at an upper and a lower portion of the rear portions (6a; 6b) of the shells (2a; 2b) and respectively provided with a rear length adjustment system (28a; 28b).

15. Corset according to any of the preceding claims, configured so as not to exert rear thrust on the spinal column.

## Patentansprüche

1. Orthopädisches Korrekturkorsett zur Korrektur von pathologischen Abweichungen der Wirbelsäule eines Patienten/einer Patientin; das Korsett (1) umfasst zwei Schalen (2a, 2b), die so ausgestaltet sind, dass sie entlang der jeweiligen Seiten des Rumpfes des Patienten/der Patientin angeordnet werden können, und eine Verbindungsvorrichtung (4), die so ausgestaltet ist, dass sie die Schalen (2a, 2b) miteinander verbindet;
wobei jede Schale (2a, 2b) mit einem vorderen Abschnitt (5a, 5b), der sich im Gebrauch von der jeweiligen Seite entlang des vorderen Abschnitts des Rumpfes des Patienten/der Patientin erstreckt, und einem hinteren Abschnitt (6a, 6b), der sich im Gebrauch von der jeweiligen Seite entlang des hinteren Abschnitts des Rumpfes des Patienten/der Patientin erstreckt, versehen ist;
die hinteren Abschnitte (6a, 6b) der Schalen (2a, 2b) so geformt sind, dass sie im Gebrauch ein Rückenband einschließlich der Wirbelsäule frei lassen;
wobei jeder vordere Abschnitt (5a, 5b) jeder Schale (2a, 2b) so geformt ist, dass er mindestens einen jeweiligen Thoraxabschnitt des Rumpfes des Patienten/der Patientin umschließt, **dadurch gekennzeichnet, dass** jeder vordere Abschnitt mit mindestens einer Schubvorrichtung (7a, 7b) versehen ist, die so angeordnet und konfiguriert ist, dass sie eine Schubwirkung auf den Thoraxabschnitt ausübt.

2. Korsett nach Anspruch 1, wobei der Thoraxabschnitt, auf den die Schubvorrichtung (7a, 7b) einwirkt, in Bezug auf den Brustbeinkörper seitlich angeordnet ist.

3. Korsett nach Anspruch 2, wobei der Thoraxabschnitt, auf den die Schubvorrichtung (7a, 7b) wirkt, zwischen der achten und zehnten Rippe liegt.

4. Korsett nach einem der vorhergehenden Ansprüche, wobei jede Schubvorrichtung (7a, 7b) einen Kontaktkörper (15a, 15b), der geeignet ist, im Gebrauch in Kontakt mit dem jeweiligen Thoraxabschnitt angeordnet zu werden, und eine Einstellvorrichtung (16a, 16b) umfasst, die konfiguriert ist, um den durch den Kontaktkörper (15a, 15b) auf den jeweiligen Thoraxabschnitt des Patienten/der Patientin ausgeübten Schub einzustellen.

5. Korsett nach Anspruch 4, wobei die Einstellvorrichtung (16a, 16b) so konfiguriert ist, dass sie die Position des Kontaktkörpers (15a, 15b) in Bezug auf eine Innenfläche (20a, 20b) des vorderen Abschnitts (5a, 5b) der Schale (2a, 2b) einstellt.

6. Korsett nach Anspruch 5, wobei die Einstellvorrichtung (16a, 16b) in einem Durchgangsloch (22a, 22b) des vorderen Abschnitts (5a, 5b) der Schale (2a, 2b) untergebracht ist.

7. Korsett nach einem der vorhergehenden Ansprüche, wobei der vordere Abschnitt (5a, 5b) jeder Schale (2a, 2b) mit einer ersten Kante (9a, 9b), einer zweiten Kante (10a, 10b) und einer Endkante (11a, 11b) versehen ist, die sich zwischen der ersten Kante (9a, 9b) und der zweiten Kante (10a, 10b) erstreckt; wobei die Endkante (11a, 11b) mit einem Fortsatz (12a, 12b) versehen ist, der mit der jeweiligen Schubvorrichtung (7a, 7b) versehen und so konfiguriert ist, dass er zumindest den Thoraxabschnitt, auf den die Schubvorrichtung (7a, 7b) wirkt, umschließt.

8. Korsett nach Anspruch 7, wobei der Fortsatz (12a, 12b) proximal zur zweiten Kante (10a, 10b) angeordnet ist, wenn das Korsett (1) entlang der Seiten des Patienten/der Patientin auf Brusthöhe zur Korrektur von Thoraxskoliose angeordnet ist, oder proximal zur ersten Kante (9a, 9b) angeordnet ist, wenn das Korsett (1) entlang der Seiten des Patienten/der Patientin auf Bauchhöhe zur Korrektur einer lumbalen Skoliose angeordnet ist, oder im Wesentlichen mittig zwischen der ersten Kante (9a, 9b) und der zweiten Kante (10a, 10b) angeordnet ist, wenn das Korsett (1) entlang der Seiten des Patienten/der Patientin entlang des lumbalen und thorakalen Bereichs zur Korrektur einer kombinierten Skoliose angeordnet ist.

9. Korsett nach einem der vorhergehenden Ansprüche, wobei sich der hintere Abschnitt (6a, 6b) jeder Schale (2a, 2b) von der jeweiligen Seite entlang des hinteren Abschnitts des Rumpfes des Patienten/der Patientin erstreckt, ohne sich über eine vertikale Achse (Ba, Bb) zu kreuzen, die durch den Beckenkamm proximal zu der Seite verläuft, entlang der die Schale (2a, 2b) angeordnet ist.

10. Korsett nach einem der vorhergehenden Ansprüche, wobei die Verbindungsvorrichtung (4) so konfiguriert ist, dass sie die Schalen (2a, 2b) so verbindet, dass ihr gegenseitiger Abstand begrenzt wird.

11. Korsett nach einem der vorhergehenden Ansprüche, wobei die Verbindungsvorrichtung (4) so konfiguriert ist, dass sie die Schalen verbindet, sodass die vorderen Abschnitte (5a, 5b) in einem bestimmten vorderen Abstand (DA) angeordnet sind und die hinteren Abschnitte (6a, 6b) in einem bestimmten hinteren Abstand (DP) angeordnet sind.

12. Korsett nach Anspruch 11, wobei die Verbindungsvorrichtung (4) so konfiguriert ist, dass der vordere Abstand (DA) zwischen den vorderen Abschnitten (5a, 5b) der Schalen (2a, 2b) einstellbar ist und/oder der hintere Abstand (DP) zwischen den hinteren Abschnitten (6a, 6b) der Schalen (2a, 2b) einstellbar ist.

13. Korsett nach Anspruch 12, wobei die Verbindungsvorrichtung (4) mindestens einen vorderen Verbindungsriemen (25), der mit einem vorderen Längenverstellsystem (26) versehen ist, und einen hinteren Verbindungsriemen (27), der mit einem hinteren Längenverstellsystem (28) versehen ist, umfasst.

14. Korsett nach Anspruch 13, wobei die Verbindungsvorrichtung (4) mindestens zwei vordere Verbindungsriemen (25a; 25b), die jeweils an einem oberen und einem unteren Abschnitt der vorderen Abschnitte (5a; 5b) der Schalen (2a; 2b) angeordnet und jeweils mit einem vorderen Längenverstellsystem (27a; 27b) versehen sind, und/oder mindestens zwei hintere Verbindungsriemen (26a; 26b) umfasst, die jeweils an einem oberen und einem unteren Abschnitt der hinteren Abschnitte (6a; 6b) der Schalen (2a; 2b) angeordnet und jeweils mit einem hinteren Längenverstellsystem (28a; 28b) versehen sind.

15. Korsett nach einem der vorhergehenden Ansprüche, das so konfiguriert ist, dass es keinen Rückwärtsschub auf die Wirbelsäule ausübt.

## Revendications

1. Corset de correction orthopédique pour la correction des déviations pathologiques de la colonne vertébrale d'un patient ; le corset (1) comprend deux coques (2a, 2b), conçues pour être disposées le long des côtés respectifs du torse du patient, et un dispositif de liaison (4), conçu pour relier les coques (2a, 2b) l'une à l'autre ;
chaque coque (2a, 2b) est pourvue d'une partie avant (5a, 5b) qui, en cours d'utilisation, s'étend du côté respectif le long de la partie avant du torse du patient, et d'une partie arrière (6a, 6b) qui, en cours d'utilisation, s'étend du côté respectif le long de la partie arrière du torse du patient ;
les parties arrière (6a, 6b) des coques (2a, 2b) sont façonnées de manière à laisser libre, en cours d'utilisation, une bande dorsale comprenant la colonne vertébrale ;
chaque partie avant (5a, 5b) de chaque coque (2a, 2b) étant façonnée de manière à sous-tendre au moins une partie thoracique respective du torse du patient, **caractérisé en ce que** chaque partie avant est pourvue d'au moins un dispositif de poussée (7a, 7b) agencé et configuré pour exercer une action de poussée sur ladite partie thoracique.

2. Corset selon la revendication 1, dans lequel la partie thoracique sur laquelle le dispositif de poussée (7a, 7b) agit est disposée latéralement par rapport au corps du sternum.

3. Corset selon la revendication 2, dans lequel la partie thoracique sur laquelle le dispositif de poussée (7a, 7b) agit est entre la huitième et la dixième côte.

4. Corset selon l'une quelconque des revendications précédentes, dans lequel chaque dispositif de poussée (7a, 7b) comprend un corps de contact (15a, 15b), apte à être disposé, en cours d'utilisation, au contact de la partie thoracique respective, et un dispositif de réglage (16a, 16b), configuré pour régler la poussée exercée par le corps de contact (15a, 15b) sur la partie thoracique respective du patient.

5. Corset selon la revendication 4, dans lequel le dispositif de réglage (16a, 16b) est configuré pour régler la position du corps de contact (15a, 15b) par rapport à une face interne (20a, 20b) de la partie avant (5a, 5b) de la coque (2a, 2b).

6. Corset selon la revendication 5, dans lequel le dispositif de réglage (16a, 16b) est logé dans un trou traversant (22a, 22b) de la partie avant (5a, 5b) de la coque (2a, 2b).

7. Corset selon l'une quelconque des revendications précédentes, dans lequel la partie avant (5a, 5b) de chaque coque (2a, 2b) est pourvue d'un premier bord (9a, 9b), d'un second bord (10a, 10b) et d'un bord terminal (11a, 11b) s'étendant entre le premier bord (9a, 9b) et le second bord (10a, 10b) ; le bord terminal (11a, 11b) étant pourvu d'un appendice (12a, 12b) fourni avec le dispositif de poussée respectif (7a, 7b) et configuré de manière à sous-tendre au moins la partie thoracique sur laquelle le dispositif de poussée (7a, 7b) agit.

8. Corset selon la revendication 7, dans lequel l'appendice (12a, 12b) est disposé à proximité du second bord (10a, 10b) lorsque le corset (1) est disposé le long des flancs du patient à hauteur thoracique pour la correction de la scoliose thoracique ou est disposé à proximité du premier bord (9a, 9b) lorsque le corset (1) est disposé le long des flancs du patient à hauteur abdominale pour la correction d'une scoliose lombaire ou est disposé sensiblement au centre entre le premier bord (9a, 9b) et le second bord (10a, 10b) lorsque le corset (1) est disposé le long des flancs du patient dans la zone lombaire et thoracique pour la correction d'une scoliose combinée.

9. Corset selon l'une quelconque des revendications précédentes, dans lequel la partie arrière (6a, 6b) de chaque coque (2a, 2b) s'étend depuis le côté respectif le long de la partie arrière du torse du patient sans passer par un axe vertical (Ba, Bb) passant par la crête iliaque proximale au côté le long duquel la coque (2a, 2b) est disposée.

10. Corset selon l'une quelconque des revendications précédentes, dans lequel le dispositif de liaison (4) est configuré pour relier les coques (2a, 2b) de manière à limiter leur éloignement mutuel.

11. Corset selon l'une quelconque des revendications précédentes, dans lequel le dispositif de liaison (4) est configuré pour relier les coques de sorte que les parties avant (5a, 5b) soient disposées à une certaine distance avant (DA) et que les parties arrière (6a, 6b) soient disposées à une certaine distance arrière (DP).

12. Corset selon la revendication 11, dans lequel le dispositif de liaison (4) est configuré de sorte que la distance avant (DA) entre les parties avant (5a, 5b) des coques (2a, 2b) soit réglable et/ou que la distance arrière (DP) entre les parties arrière (6a, 6b) des coques (2a, 2b) soit réglable.

13. Corset selon la revendication 12, dans lequel le dispositif de liaison (4) comprend au moins une sangle de liaison avant (25) pourvue d'un système de réglage de la longueur avant (26) et une sangle de liaison arrière (27) pourvue d'un système de réglage de la longueur arrière (28).

14. Corset selon la revendication 13, dans lequel le dispositif de liaison (4) comprend au moins deux sangles de liaison avant (25a ; 25b), respectivement disposées en haut et en bas des parties avant (5a ; 5b) des coques (2a ; 2b) et respectivement pourvues d'un système de réglage de la longueur avant (27a ; 27b), et/ou au moins deux sangles de liaison arrière (26a ; 26b), disposées respectivement en haut et en bas des parties arrière (6a ; 6b) des coques (2a ; 2b) et pourvues respectivement d'un système de réglage de la longueur arrière (28a ; 28b).

15. Corset selon l'une quelconque des revendications précédentes, configuré pour ne pas exercer de poussée arrière sur la colonne vertébrale.
